# EUROPEAN PATENT APPLICATION

(11) **EP 2 333 111 A1**
(43) Date of publication of application: **15.06.2011**
(21) Application number: 09755067.7
(22) Date of filing: 22.05.2009
(51) Int. Cl.: C12Q 1/68, C12P 19/34

(54) **TJN MOLECULAR KIT FOR DIAGNOSING VIRULENT STRAINS OF HELICOBACTER PYLORI**

(30) Priority: 26.05.2008 CL 15202008
(71) Applicant: Universidad De Concepcion, Santiago (CL)
(72) Inventor: GARCÍA CANCINO, Apolinaria, Concepción Santiago (CL); GONZALEZ CORREA, Carlos, Concepción Santiago (CL); TRABAL FERNÁNDEZ, Natalia, La Paz Baja California Sur (MX)
(74) Representative: Carvajal y Urquijo, Isabel
(86) International application number: PCT/MX2009/000047
(87) International publication number: WO 2009/145603

(57) **Abstract**

The invention is a procedure to detect the virulence of *Helicobacter pylori* strains wherein the procedure comprises the stages of a DNA extraction, sequence amplification through multiple PCR, using determined primers and visualization of the amplified products.

## Description

### BACKGROUND

Infections caused by *Helicobacter pylori* are a major public health problem, as they are expensive to control. This situation has led to the need for tools to detect risk factors related to this pathology. However, at the present time there are no commercially available microbiological examinations that detect virulent strains.

Because there are at present no laboratory tools for the reliable detection of H. *pylori,* eradication treatments for this bacterium are based solely on a qualitative method, and there are no treatments or therapies to determine the virulence of *H*. *pylori.*

Existing methods only indicate the presence or absence of the microorganism, but do not reveal the properties or characteristics of the infecting strain. Another important aspect is that endoscopic studies in the child population are carried out very sporadically, so that little infection data exists for this age group.

According to world-wide reports, eradication therapies fail in 20% to 80% of cases for different reasons, including the emergence of strains resistant to antibacterial agents and the absence of susceptibility studies preceding choice of treatment. The situation described implies a high health cost for the patient and for the health system. The lack of clear information with respect to the pathogenic potential of the infecting strain hinders the recovery of the patient.

Classically this bacterium has been identified at a generic level through the reaction of the urease enzyme, which allows the colonization of gastric mucosa, since in the presence of urea, hydrogen ions and water it catalyzes the formation of ammonium and bicarbonate, which neutralizes the hydrogen ions that surround the bacterium and allow its survival in the gastric epithelium (Hazell et al, 1986; Mobley et al, 1988; Megraud et al, 1989).

*H. pylori* possesses different virulence factors, which allows it to colonize stomach gastric mucosa and free itself from the defense mechanisms of the host. Some of these factors are characteristic of the species and others are of variable presence. Among the classical virulence factors present in all strains are urease, spiral structure, flagella and adhesins, as well as endotoxin (LPS); in addition, the arginase enzyme participates in the evasion of the immune response. The adhesins expressed by the bacterium recognize specific sugars from the epithelial cell. The most widely studied are N-acetyl neuraminillactose hemagglutinin and the protein BabA (*blood group antigen binding adhesin*), the latter permitting the binding of the bacterium to the Lewis Le^{B} group antigens in gastric mucosa.

Colonization by this bacterium is helped by the presence of specific proteins, such as lipases (mucinases) that degrade the mucus altering the hydrophobicity of the epithelial surface. This makes movement possible in the gastric mucosa and the bacterial proteases hydrolyze the immunoglobulins of the host (Smoot, 1997). At the same time, the bacterium avoids immunitary reaction, due to the presence of a specific protease of the IgA secretor and additionally the LPS has low immunogenic power, which inhibits an effective immunitary reaction from the host (Muotiala et al, 1992).

Different genes have been studied in an attempt to establish which strains of *H. pylori* are more pathogenic and if there is a relationship between them and the clinical manifestations of the infection. It is known in the art that virulence depends on a great number of genes, most of which reside in a pathogenicity island (PAI). The most widely studied genes are *vacA and cagA, iceA, babA2 and dupA* (Zambon et al, 2003; Faúndez et al, 2002; Censini et al, 1996). Some authors have suggested an analysis of combinations of these markers; for example, an analysis of *vacA* and *cagA* together. In this respect, *H. pylori* strains with a signal sequence of type s1 *vacA* are associated with greater gastric inflammation and with peptic ulcer illness, while *vacA* alleles that are type m1 middle region are related to more severe epithelial damage (Pan et al, 1999). Other researchers suggest that *cagA* and *iceA* genotypes would be a good combination of markers for the identification of patients with peptic ulcers (Faúndez et al, 2002).

A new gene that has been involved in the pathogenesis of *H pylori* is the gene that codifies for the proteins BabA1 and BabA2, which allow the activation and deactivation of synthesis of adhesins (Gerhard et al, 1996).

A search has been carried out in the main data bases and patent offices in the world. The documents that are most closely related to the present invention are discussed in the following section.

A product based on the microarray technique of MWG Biotech, Inc. designed to detect *H. pylori* is available on the market. This product consists of 1877 oligonucleotideees for recognizing *H. pylori* strains J99 and 26695. 1307 of these oligonucleotideees coincide in both strains, there being 295 specific oligonucleotideees of *H*. *pylori* strain 26695 and 278 specific oligonucleotideees of *H*. *pylori* strain J99. There are no related invention patent applications.

The company Invitek developed a kit, commercially named INVIGENEE®, for the detection of the genotype *cagA* of *H*. *pylori* in feces, capable of determining the presence of active infection of this bacterium. This device is not related to an invention patent application. This device only makes it possible to establish the presence of the microorganism, not its virulence. The product "MPCR KIT for *H*. *pylori* detection" and the MP-70080 of the company Maxim Biotech, Inc have been developed using multiple PCR technique. The kit is designed to rapidly detect *H*. *pylori* using multiple PCR. The primers contained in the kit amplify the genes *cagA* (358 bp), flagellin (152bp), *urea C* (315bp) and ARNr 16S (110bp). The product includes all the reactives necessary for amplification, with the exception of the enzyme polymerase Taq and dNTPs. The kit includes the work buffer, the respective positive control, the molecular weight marker, water free from DNAases and specific primers. Neither the primers nor the genes used in this kit are used in our invention.

There are 64 invention patent applications related to the present invention. Detailed information about the applications and existing patents most closely related to the *H*. *pylori* detection kit now follows:

Inventions related to the kit with regard to the detection of urease.

**Table 1. Related inventions with H pylori detection kits**

| Patent number | Determines Virulence | Tissue collected | Particle detected | Tissue samples | Molecule identified |
|---|---|---|---|---|---|
| JP2006284567 | Yes | No tissues collected | Ig | Urine or saliva | Urease/ protein flagellar/VacA/Cag A/NapA |
| TW24780913 | No | No | Monoclonal Ig | Feces | *H. pylori* catalase |
| KR20000033013 | * | * | * | Not determined | Urease |
| W09951769 | No | No | No | Biopsies | Chemical reactions |
| AU2002355464 | No | Blood | C13 | Blood sample | Urea marked with C13, increase in absorbency |
| KR20030031243 | * | * | Primers VacA, CAG, among others | * | VacA, CagA, RT-PCR technique used |
| US2006171887 | No | Blood or breath | Urea marked in the form of acid | Breath or blood | Urea |
| EP1685851 | No | Breath | Urea marked C13 in the form of acid | Breath or blood | Generic identification |
| US2006133999 | No | Blood from finger or earlobe | C13 marked urea, an acid ointment taken | Blood samples | Peripheric carbon |
| JP2006075139 | Yes | * | Specific nucleotideeic sequences | * | cagA gene |
| WO2005108995 | Yes | Blood | Ig | Blood, serum | HP1, HP2 and HP3 |
| CN1687134 | NOT determined | Not divulged | Ig | Not known | HpaA and urease B |
| WO2004111265 | No | Biopsy | Urease in the sample | Stomach biopsy | *H pylori* Urease |
| US2005048077 | No | Blood | Ig | Serum | HP1, HP2 and HP3 proteins of *H. pylori* |
| EP1156331 | No | Feces | Reaction with Ig | Feces | Alkaline phosphatase and beta galactosidase and horseradish peroxidase. |
| CN1465980 | No | Not obtained | *H pylori* | Indeterminate | *H. pylori* cell membrane antigens |
| WO2004040306 | No | Blood | Ig | Serum | An *H. pylori* membrane protein |
| WO02088737 | No | Feces | Ig | Serum | *H. pylori* catalase |
| WO03080840 | Not known | Not determined | Ig | Serum | Antigens of related polymorphic genes |
| WO02054084 | No | Serum, urine, tears and/or saliva | Ig | Serum | *H. pylori* pepsinogen type I and gastrin recognized |
| WO0214541 | No | Feces or stomach samples | Ig | Serum | *H. pylori* catalase recognized |
| WO0192889 | No | Breath | C marked | Breath, exhaled air | A proportion of the marked carbon that is associated with the exhaled CO₂ and is titered using the bacteria-fixed carbon |
| JP2002119280 | No | Blood | *H pylori* | Blood, serum | *H. pylori* through a monoclonal Ig |
| WO0029618 | Yes | Biopsy | *H. pylori vac A* or *cagA* genes | From digestive tract | *H. pylori vac A* or *cagA* genes |
| US6171811 | No | Breath | *H pylori* indirectly | Breath | Administer citric acid with marked C13. |
| JP2000321271 | Only presence or absence of *H pylori* | Exhaled air | Ammonium substitution | Exhaled air | Ammonium reaction, the presence of *H*. *pylori* is determined according to the relationship with exhaled gases |
| WO9932656 | No | Gastric mucosa | *H. pylori* | Gastric mucosa | Urea, an indirect calorimetric system to determine *H pylori* in the medium |
| DE19847628 | No | Blood, detects Ig | *H. pylori* surface protein | Blood | *H. pylori* protein |
| US5955054 | No | Not clearly determined | *H pylori* using a gamma camera provided with an ionization detector | Not determined if obtained from feces or saliva, if a non-invasive method | *H pylori* using an ionization method that detects technetium 99 differentially |
| WO9949890 | Yes, determines the presence of ad-hesins | Can be blood, if it is an immuno-logical method | *H. pylori*, a specific adhesin | Types of samples not established in the claims | Adhesin of *H. pylori*/CTXA2B |
| US5981184 | No | *H. pylori* | *H. pylori* ATPase | Not determined how *H. Pylori* obtained | *H. pylori* ATPase |
| WO9853082 | Yes | Stomach sample | *H. pylori* | Stomach sample determined how obtained | IgA adhesins |
| WO9612965 | No | Serum in the blood | *H. pylori,* an antigen | Blood | An antigen immobilized in a support membrane |
| US5846751 | No | Serum | *H pylori* | "Biological" sample | *H. pylori* serological assays such as ELISA, latex agglutination, and rapid EIA |

| | | | | | |
|---|---|---|---|---|---|
| ** No information given* | | | | | |

The invention is different from other initiatives, because the invented kit detects prevalent genes associated with virulence. Commercial kits and other related inventions generally detect the presence of the enzyme urease (characteristic of *Helicobacter sp.*), using a visualized positive test using pH indicator toning, which does not necessarily indicate that it is an *H. pylori* strain, much less that it may be a potentially pathogenic strain.

In addition, most of the existing kits are not specific, as other helicobacteria present in the biopsy, such as the species *H. helmannii* for example, also generate a positive urease reaction, this being a characteristic associated with the *Helicobacter* genus. Other kits used are based on the detection of antibodies against the bacterium in serum.

Our search shows that there are 7 closely-related invention patent applications, as follows: JP2006284567, KR20030031243, WO2005108995,

WO2004111265, WO0029618, WO9949890 and WO9853082. The most important of these invention patent applications are the patents KR20030031243 and WO2005108995, which are directly related to the present invention, because the said initiatives determine the toxicity of *H. pylori* at a genetic level. However, none of those patents associate more than 2 virulence genes, at the most an identification sequence, or they are difficult techniques to implement.

No initiatives with similar characteristics to the proposed invention have been found and, still more importantly, the particularities of the above initiatives have been developed from strains whose gene sequences are not related to the sequences that are being claimed in the present invention.

### BRIEF DESCRIPTION OF THE INVENTION

This molecular kit is different from existing initiatives both in the genes it recognizes and in the reactives it requires. Existing initiatives take more time to find optimal conditions to simultaneously amplify genes associated with virulence in the above-mentioned existing initiatives that determine them.

Other inventions have the disadvantage that the DNA concentration of some biopsy samples does not allow the simultaneous detection of a number of genes, unlike the present invention. There is no known kit that detects a variety of virulence factors, so that this invention offers advantages in design and innovation with respect to existing kits. This invention can generate a genetic pattern in *H*. *pylori* associated with more severe pathologies, which is an aspect not included in any previous initiative.

The molecular kit for the simultaneous detection of genes associated with virulence and the species allows the recognition of *H*. *pylori* strains with a greater capacity for colonization and human infection, which are associated with severe pathologies derived from chronic *H*. *pylori* infection. This kit permits rapid and efficient detection of *H*. *pylori* strains with greater pathogenic potential.

The invented product is clearly different from and superior to existing methods of diagnosis, since at the present time there are no commercial products that detect *H*. *pylori* genes from specific strains, and more importantly, the few existing products include at the most only one gene associated with virulence and another with specificity.

### DESCRIPTION OF THE INVENTION

The invention is related to a kit for diagnosing infection caused by *H*. *pylori* that requires human samples. Subsequently, the kit is applied in a preestablished protocol.

This invention kit is conceived for the genetic detection of *H. pylori.* This initiative determines the existence of a number of different virulence genes of these microorganisms, associated with specific sequences. In addition, there is no existing initiative that relates a number of identification and virulence genes at the same time for diagnosis. Even if there are other technologies that are similar with regard to their objectives, that is, to detect this microorganism, there are few technologies aimed at determining the existence of a number of different virulence genes of these microorganisms.

There is no initiative in existence that relates identification and virulence genes at the same time for diagnosis, all of which are associated with the specific sequences that are divulged in the present invention.

An important advantage of this kit is that it contains all the elements for its optimal performance, such as Taq polymerase, dNTPs, work buffer, respective positive control, molecular weight marker, specific primers and water free from DNAase. In addition, it presents the protocols necessary for DNA extraction. These are innovatory aspects of insuperable value in this initiative, not considered in similar inventions.

In the art, there are no products that detect various *H*. *pylori* virulence genes and at the same time recognize genes characteristic of the *H*. *pylori* species. What is more, a great number of inventions claim kits whose only application is oriented to the recognition of specific strains, recognizing at the most two genes present in the species. What is surprising and unexpected in this invention is the combination of genes selected. In addition this kit is designed to be applied in any part of the world and to diverse types of samples.

This kit has the particularity that it can be used for the detection of strains whose distribution is very wide, making it possible to extend the applications of the invention with regard to where virulence indices and their range can be determined. In the invention, work has been done with a group of *H*. *pylori* genes with high incidence, and a kit has been created based on sequences of strains that are representative of extensive geographical areas. Thus the present initiative is highly significant for a variety of ethnic groups and responds to an age-old problem in the diagnosis of highly pathogenic *H. pylori* that has remained without a solution until now. This technology has overcome previous obstacles to create a solution that can be widely applied.

Another advantageous aspect of this technology is its extreme reliability; the probability of obtaining consistent and certain results is without doubt very high. Although these are attributes possessed by any test or kit with a molecular base, in this case it is only right to point out that the rigorous selection of the genetic sequences to replicate are determinant in obtaining precise results, and the fact that it uses a large number of these genetic sequences gives this technology a clear advantage over other tests.

In addition, this initiative presents the advantage that these results are unexpectedly exact, because of detection with regard to a number of genes that when evaluated together offer revealing evidence of *H*. *pylori* virulence. Therefore this analytical diagnosis technology is far superior to the existing inventions.

Another distinctive characteristic of this invention is its clinical validity; that is, the certainty with which this DNA kit gives a diagnosis, predicting the risk of an illness in clinical practice. The clinical validity of this kit offers a test that includes reactives providing unusual sensitivity. The positive prediction value, in other words, the probability that the persons with positive results for this test will develop a related pathology, and the negative prediction value associated with this tool, that is the probability that the persons with negative results will not develop the illness, is an aspect that this kit considers, which has not been considered in similar inventions, again giving it clear superiority over existing kits. The kit can detect not only the presence of an *H. pylori* strain, but also, surprisingly, the virulence genes possessed by the bacterium. A molecular kit with all these advantages was developed based on simultaneous detection of genes associated with virulence in *H. pylori: cagA, vacAm1,* and *dupA,* which allows the recognition of strains with greater pathogenic potential.

The procedure for the detection of this organism is carried out on samples from patients in which this microorganism is found or part of its genetic material. It is preferable to use biopsies from the intestinal tract, feces, blood, serum, breath, among others, in which the *H. pylori* strains with greatest pathogenic potential are detected rapidly and effectively. This new tool makes it possible for the doctor to take decisions with regard to the therapeutic management of the patient infected by *H*. *pylori.*

Traditional microbiological and molecular diagnosis of *H. pylori* generally has the disadvantage that personnel must be trained in diagnostic procedures in endosonagraphy, endoscopy and hystopathology. With this kit, however, these technical difficulties do not exist because the kit is easy to use. The introduction of the diagnostic kit into clinical routine constitutes a health saving, since it permits early detection of infection by pathogenic strains, especially among children, which will prevent the progression of the infection to further damage.

Another aspect covered by this invention that most existing kits have not solved is the determination of the virulence of strains. Most related inventions are designed for clinical use and not prevention. This invention solves a permanent problem in the art, that of the detection of virulent strains. Above all, this kit allows the detection of virulent strains even when the person has no clinical symptomatology related to suffering from the pathology that is directly correlated to the virulent microorganism.

This invention can be implemented in any clinical diagnosis laboratory and requires only basic personnel training and simple equipment. This kit is a real alternative to traditional culture in microbiological diagnosis laboratories, with the advantage that it saves time and materials. It provides more information for the doctor than the technologies currently available on the market, since it allows the rapid recognition of *H*. *pylori* strains with the greatest pathogenic potential.

Knowing that one is facing a pathogenic strain also makes it possible to take appropriate action to prevent the appearance of severe illnesses and to recover the health of the person, which improves the quality of life of the population with gastroduodenal pathology associated with *H. pylori.*

Another advantage of this kit is that it can be adapted for use in PCR equipment in real time, which guarantees greater sensitivity, since it detects quantities as small as 3pg of DNA, even if the DNA is diluted to limit levels. In addition, this technique considerably shortens the time taken to obtain a result.

Another operating advantage of this invention is that it works through multiple PCR. The only existing related invention that uses multiple PCR requires the addition of some fresh reactives; this invention, however, only needs distilled water free of nucleases, or it can be presented in both forms. In addition, the virulence genes detected by the kit in the present invention are genes with high incidence

The proposed molecular examination shortens the time taken to obtain the result, since a traditional culture needs at least one week. This technique is therefore the best current option for diagnosing pathogenic *H*. *pylori.* This invention has short, medium and long term advantages, so that it can increase the quality of life of people, improving their health and slowing the advance of chronic *H*. *pylori* infection to more severe gastroduodenal pathologies, with an important impact on the diagnosis and treatment of the pathologies caused by *H*. *pylori.*

The development-of a molecular kit for the simultaneous detection of genes associated with virulence in *H. pylori* (*cagA*, *vacAm1,* and *dupA*) prevalent in the population, which allows the recognition of strains with greatest capacity for colonization, chronic infection and production of damage to human gastric mucosa has not previously been claimed.

The present invention now makes it possible to determine the prevalence of *H. pylori* strains present in different samples, preferably in gastric biopsies with different gastroduodenal pathologies, or without symptomatology related to this microorganism. In addition, this invention determines tiny concentrations of DNA (1µg/µl), which are sufficient to detect *H*. *pylori.* The proposed initiative determines the optimal conditions for simultaneously amplifying two or more genes associated with virulence in strains of *H. pylori* present in samples of feces, fluids or gastric biopsies.

Until now, the doctor has not had bacteriological laboratory support available for decisions with regard to timing of eradication treatment of *H. pylori* and choice of the most suitable therapy to increase the probabilities of successful eradication. The doctor uses the urease test to detect the bacterium, for example, based on the gastric sample. However, a positive reaction only indicates the presence of *Helicobacter sp.;* it is not specie specific and much less indicates the pathogenic potential.

The absence of effective and accessible methods for identifying populations of high risk and of useful biological markers for early diagnosis, are valid considerations in the solution of this problem and these considerations are taken into account in the solution divulged in this document.

The use of multiple PCR allows the rapid and efficient detection of *H*. *pylori* strains with greater pathogenic potential based on samples.

### DETAILED DESCRIPTION OF THE INVENTION

Specifically, this invention contemplates a process and a product that make it possible, using a number of analysis steps, to identify a microorganism present in a sample, in addition to determining the virulence of the specific strain.

The processing order is the following:

Once the sample has been obtained, the DNA is extracted, according to the specific recommendations in the kit, which ensures a good result with regard to the quality and quantity of the DNA extracted. The total DNA from gastric biopsies, pure cultures, body fluids or feces is in general terms based on the treatment of the sample with proteinase K followed by alcoholic precipitation (with ethanol-chloroform or isopropanol).

The genotypification of *H*. *pylori* strains through multiple PCR consists in the amplification of various genes at the same time, which is why lyophilized PCR spheres that contain all the reactives necessary for amplification (dNTPs, Taq polymerase, specific primers, reaction buffer, MgCl₂), which must be reconstituted with the appropriate quantity of water to the final volume in a range of 15 - 30µL. The hybridization temperature range required is from 40 to 55 °C and 30 to 45 PCR cycles are used. As an initial condition, ready-to-use PCR beads are used (Amersham Biosciences®) in a range of 0.1 to 10 ng/µL DNA (previously extracted).

Finally, amplification is carried out in a thermal cycler with programmable control, according to the following program:
- The procedure implies initial denaturation at a temperature ranging from 80 to 98 °C, for a period of time between 1 and 15 minutes,
- then soft denaturation, using the same temperature range, for a period of time between 0.1 and 3mins,
- subsequently, hybridization is carried out at a temperature ranging from 45 to 60 °C for a period of time between 1 and 70secs,
- the initial extension stage is carried out at a temperature of between 65 and 75 °C for 60 seconds,
- the final extension stage is carried out at the same range of temperatures as the initial extension, but the period of time is from 3 to 10 mins.

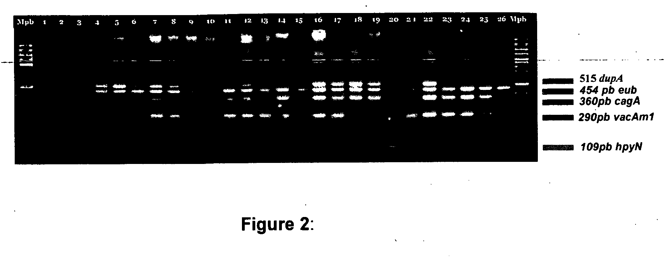

The kit contains negative controls that can be used in all the stages, particularly during amplification (human genome DNA). It also has positive controls (*H*. *pylori* DNA strain ATCC 43504), which are a DNA sequence as a template; these controls are run in parallel. The products of this amplification are analyzed using agarose gel electrophoresis at 3% (w/v) and stained with ethidium bromide (0.5 µg/µL, or sybr green, Figure 2). The positive controls used are the multiple PCR amplification pattern for the genes *cagA* and *vacAm1* from the genomic DNA, derived from *H. pylori* ATCC43504 control strains. The amplified fragments are analyzed using agarose gel electrophoresis at 3%, followed by staining with ethidium bromide and visualization with a UV transilluminator.

If the DNA content is not sufficient to amplify all the genes desired, the invention solves this problem by using the culture duplicate samples, which is an aspect that has been considered in this particular protocol. Nevertheless, the invention demonstrates that small quantities of DNA (1ng/µL) are enough to amplify various genes, either simultaneously or separately, without any problems.

The invention includes a kit that is a ready-to-use product, capable of detecting 5 genes simultaneously: one *Helicobacter pylori* identification gene, three virulence genes; *vacAm1, cagA* and *dupA,* and a pair of primers that determine the quality of DNA- extraction (universal eubacterial gene 16-23S).

The invention also includes a kit and a procedure that considers reactives and stages and therefore has the following elements:
I. DNA extraction from samples stage. This is optional, according to the type of sample treated.
II. Reactives and PCR multiple amplification stage. Every sample that has already been processed and whose result ends in pure DNA is subjected to this stage.

The kit includes an amplification reactive to carry out the multiple PCR technique and the positive and negative controls.

### PCR reactives

A reaction buffer, which is Tris-HCl, pH range between 8.5 and 9.5, KCI in a range of concentrations between 480 and 560 mM and MgCl₂ in a range of concentrations between 10 and 20 mM, the thermostable enzyme Taq polymerase in a range of 400 to 6000U, MgCL²⁺, dNTPs (2'-deoxynucleosid 5'-triphosohate, containing 4 dinucleotides dATP, dCTP, dGTP, dTTP, in a range of concentrations between 15 and 30mM), and specific primers (see Table 2).

All these reactives can be found in two forms in the kit: dehydrated, sterile and lyophilized, or alternately liquid and sterile. Optionally, the kit can include H₂O of the quality needed for molecular biology tests. The kit also includes positive and negative DNA controls, obtained from pure cultures of *H*. *pylori* strains from the collection ATCC43504 and the human genome, respectively.

**Table 2. Sequence list of the primers used for the detection of Helicobacter pylori virulence and recognition genes.**

| Gene detected | Sequence | Amplification size |
|---|---|---|
| | *H. pylori* identification: | |
| *16 DNAr H. pylori* | Forward 5' CTG GAG AGA CTA AGC CCT CCA 3' | 109 pb |
| | Reverse 5' CAT TAC TGA CGC TGA TTG 3' | |
| | Virulence genes: | |
| *cagA* | Forward 5' TCA GA AAT TTG GGG ATC AGC 3' | 375 pb approx |
| | Reverse 5' GGG GAA CTG GTT CTT GAT TG 3' | |
| | Optionally: | |
| | Forward: 5' ACG ATA GGG ATA ACA GGC AAG C 3' | 360-380pb |
| | Reverse: 5'GAT CCG TTC GGAT TTG ATT CCC 3' | |
| | Optionally: | |
| | Forward: 5' TCAGAAATTTGGGGATCAGC 3' | |
| | Reverse: 5' ACATGGGGAACTGGTTCTTG 3' | 380pb |
| *vacAm alelo1* | Forward 5' ATT TGG TCC GAG GTG GGA AAG T 3' | 250 pb |
| | Reverse 5' GCT AGG CGC TCT TTG AAT TGC T 3' | |
| | Optionally: | |
| | Forward: 5' GCA ATG CAG CAG CTA TGA TG 3' | 205 pb |
| | Reverse: 5' GCG CTC TTT GAA TTG CTC TT 3' | |
| | Optionally: | |
| | Forward 5' GCA ATG CAG CAG CTA TGA TG 3' | |
| | Reverse: 5' TAG GCG CTC TTT GAA TTG CT 3' | |
| | | 209 |
| *dupA* | Forward 5' ACA AGG ACG ATT GAG CGA TGG GAA 3' | 515 pb |
| | Reverse 5' TGG CTA GTT TGA GGT CTT AGG CGT 3" | |
| | DNA quality determination: | |
| *16S DNAr Eub* | Forward 5' GCA CAA GCG GTG GAG CAT GTG G 3" | 415 pb |
| | Reverse 5' GCC CGG GAA CGT ATT CAC CG 3" | |

### III. Visualization stage of the genes detected and interpretation of data

This stage includes the preparation of a 3% agarose gel, which is an option that can be included in the kit, in addition to the specific molecular weight marker.

The sample visualization is carried out using a reactive that produces the DNA differential staining, for example, ethidium bromide, and subsequent exposure to ultraviolet light. The kit indicates the DNA staining method and can include other methods of DNA staining with ethidium bromide or alternatives such as sybr green or similar, since the staining method is nonlimiting for this invention. In addition, the kit has a simple users' manual.

### EXAMPLES

### Procedure

The kit can simultaneously detect at least four genes, one *Helicobacter pylori* identification gene and three virulence genes *vacAm1, cagA* and *dupA.* In addition, the kit involves the association of primers that determine the quality of DNA extraction. The kit has the protocols necessary for correct DNA extraction from *H. pylori* samples and/or pure cultures.

The kit is provided with the reactives necessary for multiple PCR analysis, with the use of sterile lyophilized spheres or sterile liquids, as well as the molecular weight marker and optionally can include agarose to make the gel and the revelators for gene visualization, for example, ethidium bromide or sybr green.

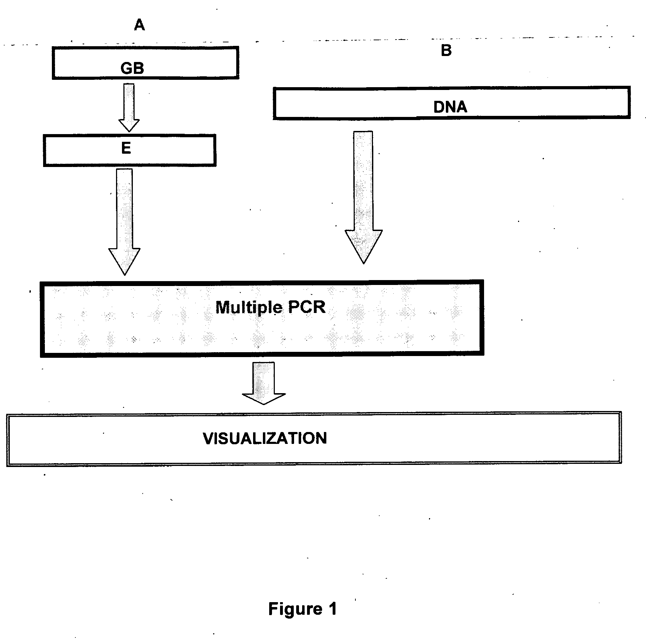

The general procedure can be seen in Figure 1, which shows a general scheme of the use of the invention kit, in which the variables are:
■ Line A is the procedure to follow when using a sample from which the DNA must be extracted, for example, GB is Gastric Biopsy,
■ E is the DNA extraction stage,
■ Line B is the application of the same procedure when the pure *H. pylori* strain has been isolated,
■ DNA is the DNA extracted from samples containing *H*. *pylori,*
■ Multiple PCR is the stage in which samples are subjected to multiple PCR. This stage is common to those samples that have been obtained through isolation of DNA from gastric biopsy samples, feces or those samples in which DNA is obtained from the directly isolated bacterium. The objective of this stage is the identification and detection of *H*. *pylori virulence genes,*
■ Visualization is the last stage of the process in which results are obtained using agarose gel electrophoresis, followed by staining and interpretation of the results.

### Example 1: Application of the kit to gastric biopsy samples

This is not a limiting example of the technology, only a particular application of the kit developed.

### Samples:

56 biopsies were analyzed from the antrum and body portions of the stomach of 26 patients who consulted a doctor about gastroduodenal problems. A sample of *H*. *pylori* strains ATCC 43504, ATCC 25695 and 96.978 was used for positive controls, and a sample of human genome DNA was used as a negative control.

### 1.- First DNA extraction stage and substages:

DNA extraction was carried out using the reactives described as follows, available in the proposed kit: lysis buffer (TrisHCl 10mM, EDTA 1mM, SDS 10%); Proteinase K (20mg/mL); CTAB/NaCl solution; Chloroform: isoamyl alcohol (24:1); phenol: chloroform: isoamyl alcohol (25:24:1); ethanol and TE buffer 50 of the biopsies analyzed were included in the trial; other samples were used as controls, from which DNA was extracted using the method of Mazurier et al, (1992) from the pure isolates from the *H*. *pylori* culture. Human genome DNA was used as a negative control.

### 2.-Second stage: H. pylori amplification using multiple PCR:

In the case of the genotypification of the invention, the DNA amplification of the *H*. *pylori* strains was carried out using multiple PCR, for which lyophilized PCR spheres were used (Lyophilised Pure Taq ready-to-go PCR Beads, Amersham; Pharmacia Biotech), which contain PCR buffer 10X (Tns-HCl 100 mM, pH 8.3, KCI 500 mM and MgCl₂ 15 mM); Taq polymerase and a concentration of 2.5U/µL, dNTPS (2'-deoxynucleosid 5'-triphosohate, containing 4 dinucleotidees dATP, dCTP, dGTP, dTTP, 10mM), MgCl₂ (100 mM) and specific primers (10pmol/L), to amplify the *H. pylori* identification genes 16S DNAr, of the quality of DNAr16S EUB and virulence genes *vacAm1, dupA* and *cagA.* All these reactives were reconstituted with water free of nucleases in a final volume of 15µL; 5µL of previously extracted DNA were added to the mixture. The hybridization temperatures varied between 50 and 74 °C and 39 amplification cycles were used.

### 3.- Third stage: visualization of the amplification products and interpretation of the data:

The results were analyzed in 3% agarose gel, 70 volts/sec for 45 mins, and were compared with a molecular weight marker, consisting of lambda phage DNA digested with HindIII digestion enzymes, which provide a profile of 12 bands of 100 pb each. Ethidium bromide was used for staining (0.5 µg/mL) and visualization was carried out using exposure to UV light. Finally, a photograph was taken. The results obtained are shown in Figure 2.

As can be seen in Figure 2, simultaneous positive amplification was obtained in all cases for the genes to be detected by the kit. In cases in which the hystopathological diagnosis of the patient was more serious, at least two of the genes involved were detected by the kit in the invention. After applying the kit, it was concluded that the patients presenting some gastroduodenal pathology are those that present the virulence genes in their gastroduodenal samples; other samples in the same trial that show the absence of these genes do not show this pathology.

### Example 2: Comparison between the invention and a commercial kit

Our technology was compared with an alternative commercial kit, MPCR Kit *H*. *pylori;* Cat N°. MP-700081. The latter showed significant differences with regard to the results, described as follows:
1) It does not contain reactives or the procedures necessary to carry out DNA extraction from gastric biopsies and/or *H. pylori* culture.
2) It does not provide the reactives Taq polymerase and dNTPS, which means that the kit user must have them ready beforehand. In addition, the reactives that it includes are in a sterile, liquid form, sensitive to loss of activity through changes in temperature (they must be stored and transported at -20 °C). Our invention includes a choice of two types of format: sterile lyophilized beads (completely stable, to be stored and transported at environmental temperature) or sterile liquid.
3) The kit MPCR Kit *H. pylori;* Cat N°. MP-700081 detects the gene *ureA,* which, according to reports in the international literature, is not exclusive to *H*. *pylori,* which means that the afore-mentioned kit only detects one virulence gene (*cagA*), while the invention proposed by us includes at least three virulence genes.
4) Unlike our invention, it does not provide a negative control.
5) Our technology includes the reactives and procedure to follow for the visualization and interpretation of the results as options.

SEQUENCE LISTING

<110>UNIVERSIDAD DE CONCEPCION, GARCIA CANCINO,
Apolinaria y otros.
<120> MOLECULAR KIT FOR DIAGNOSING VIRULENT STRAINS OF
HELICOBACTER PYLORI
<160> 18

<210> 1
<211>20
<212> ADNr
<213> H. pylori
<223> primer
<400> SEQ ID NO: 1
CTC GAG AGA CTA AGC CCT CC 20

<210> 2
<211>19
<212>ANDr
<213> H. pylori
<223> primer
<400> SEQ ID NO: 2
ATT ACT GAC GCT GAT GTG C 19

<210> 3
<211> 22
<223> primer
<400> SEQ ID NO: 3
GAT AAC AGG CAA GCT TTT GAG G 22

<210> 4
<211> 22
<223> primer
<400> SEQ ID NO: 4
CTG CAA AAG ATT GTT TGG CAG G 22

<210> 5
<211> 22
<223> primer
<400> SEQ ID NO: 5
ACG ATA GGG ATA ACA GGC AAG C 22

<210> 6
<211> 22
<223> primer
<400> SEQ ID NO: 6
GAT CCG TTC GGAT TTG ATT CCC 22

<210> 7
<211> 20
<223> primer
<400> SEQ ID NO: 7
TCA GAA ATT TGG GGA TCA GC 20

<210> 8
<211> 20
<223> primer
<400> SEQ ID NO: 8
ACA TGG GGA ACT GGT TCT TG 20

<210> 9
<211> 19
<223> primer
<400> SEQ ID NO: 9
GGT CAA AAT GCG GTC ATG G                                    19

<210> 10
<211> 20
<223> primer
<400> SEQ ID NO: 10
GTT TCT ACA GGT ACC AAT GG                                   20

<210> 11
<211> 20
<223> primer
<400> SEQ ID NO: 11
GCA ATG CAG CAG CTA TGA TG                                   20

<210> 12
<211> 20
<223> primer
<400> SEQ ID NO: 12
GCG CTC TTT GAA TTG CTC TT                                   20

<210> 13
<211> 20
<223> primer
<400> SEQ ID NO: 13
GCA ATG CAG CAG CTA TGA TG                                   20

<210> 14
<211> 20
<223> primer
<400> SEQ ID NO: 14
TAG GCG CTC TTT GAA TTG CT 20

<210> 15
<211> 21
<400> SEQ ID NO: 15
ACA AGG ACG ATT GAG CGA TGG 21

<210> 16
<211> 21
<223> primer
<400> SEQ ID NO: 16
TGG CTA GTT TGA GGT CTT AGG 21

<210> 17
<211> 22
<223> primer
<400> SEQ ID NO: 17
GCA CAA GCG GTG GAG CAT GTG G 22

<210> 18
<211> 20
<223> primer
<400> SEQ ID NO: 18
GCC CGG GAA CGT ATT CAC CG 20

## Claims

1. A procedure and kit to- detect the virulence of an *H. pylori* strain wherein the procedure comprises the following stages:
(a) DNA extraction stage,
(b) sequence amplification stage through multiple PCR, using the primers schematized in Table 1,
(c) stage of visualization and interpretation of the data.

2. A procedure and kit to detect the virulence of an *H*. *pylori* strain according to claim 1, wherein the DNA extraction stage is composed of the following phases:
i. cell lysis phase,
ii. DNA preparation phase,
iii. DNA recuperation phase.

3. A procedure and kit to detect the virulence of an *H*. *pylori* strain according to claims 1 and 2, wherein in the DNA extraction stage, specifically in the cell lysis phase, a tampon, proteinase K and CTAB/NaCl solution are used.

4. A procedure and kit to detect the virulence of an *H*. *pylori* strain according to claims 1 and 2, wherein in the DNA extraction stage, specifically the DNA preparation phase is carried out with a mixture of chloroform, isoamyl alcohol, or a mixture of phenol, chloroform and isoamyl alcohol.

5. A procedure and kit to detect the virulence of an *H. pylori* strain according to claims 1 and 2, wherein in the DNA extraction stage, specifically in the DNA recuperation phase, the amplification of the sequences of interest is carried out using the following primers:
a. *H. pylori* identification gene *16 DNAr H. pylori:*
Forward 5' CTG GAG AGA CTA AGC CCT CCA 3'
Reverse 5' CAT TAC TGA CGC TGA TTG 3',
b. Virulence genes:
*cagA*: Forward 5' TCA GA AAT TTG GGG ATC AGC 3';
Reverse: 5'GGG GAA CTG GTT CTT GAT TG 3' *vacAm allele1:* Forward 5'ATT TGG TCC GAG GTG GGA AAG T 3',
Reverse 5'GCT AGG CGC TCT TTG AAT TGC T 3'
*Gene dupA:* Forward 5' ACA AGG ACG ATT GAG CGA TGG GAA 3' Reverse 5' TGG CTA GTT TGA GGT CTT AGG CGT 3',
c. DNA quality determination gene 16S DNAr Eub:
Forward 5' GCA CAA GCG GTG GAG CAT GTG G 3', Reverse 5' GCC CGG GAA CGT ATT CAC CG 3'.

6. A procedure and kit to detect the virulence of an *H. pylori* strain according to claim 1, wherein this procedure uses positive and negative DNA extraction controls.

7. A procedure and kit to detect the virulence of an *H*. *pylori* strain according to claim 1, wherein the positive DNA extraction control is *H*. *pylori* ATCC43504 and the negative DNA extraction control is human genome.

8. A procedure and kit to detect the virulence of an *H*. *pylori* strain according to claim 1, wherein for the visualization stage an agarose gel in a range of concentrations between 1.2 and 4% must be used.

9. A kit to detect the presence and virulence of an *H*. *pylori* strain in different types of samples according to claim 1, wherein it contains the amplification reactives and the appropriate primers, in addition to the controls, optionally:
a. detection reactive and interpretation of the data,
b. cell lysis reactive,
c. precipitation reactive.

10. A kit to detect the presence and virulence of an *H*. *pylori* strain in different types of samples according to claim 9, wherein the amplification reactive contains the following elements:
a. multiple PCR reactives with the specific primers for *H*. *pylori,*
b. amplification controls of the sequences of interest.

11. A kit to detect the presence and virulence of an *H*. *pylori* strain in different types of samples according to claim 9, wherein the amplification reactive, specifically the reactives used for multiple PCR are:
a. reaction buffer
b. thermostable enzyme, Taq polymerase;
c. nucleotidees;
d. specific primers *H*. *pylori* identification gene, *16 DNAr H. pylori:*
Forward 5' CTG GAG AGA CTA AGC CCT CCA 3'; Reverse 5' CAT TAC TGA CGC TGA TTG 3';
e. virulence genes:
i. *cagA*: Forward 5' TCA GA AAT TTG GGG ATC AGC 3';
Reverse 5'GGG GAA CTG GTT CTT GAT TG 3';
ii. *vacAm allele1:* Forward 5'ATT TGG TCC GAG GTG GGA AAG T 3'; Reverse 5'G°CT AGG CGC TCT TTG AAT TGC T 3;
iii. Gene *dupA:* Forward 5' ACA AGG ACG ATT GAG CGA TGG GAA 3'; Reverse 5' TGG CTA GTT TGA GGT CTT AGG CGT 3'';
Optionally:
cagA: Forward: 5' ACG ATA GGG ATA ACA GGC AAG C 3';
Reverse: 5'GAT CCG TTC GGAT TTG ATT CCC 3';
cagA: Forward: 5' TCAGAAATTTGGGGATCAGC 3'; Reverse: 5' ACATGGGGAACTGGTTCTTG 3'.
Optionally:
*vacAm1:* Forward: 5' GCA ATG CAG CAG CTA TGA TG 3';
Reverse: 5' GCG CTC TTT GAA TTG CTC TT 3';
iv. *vacAm1:* Forward: 5' GCA ATG CAG CAG CTA TGA TG 3'; Reverse: 5' TAG GCG CTC TTT GAA TTG CT 3';
f. DNA quality determination gene 16S DNAr Eub; Forward 5' GCA CAA GCG GTG GAG CAT GTG G 3'; Reverse 5' GCC CGG GAA CGT ATT CAC CG 3''.

12. A kit to detect the presence and virulence of an *H*. *pylori* strain in different types of samples according to claim 9, wherein the reaction buffer of the amplification reactive consists of Tris-HCl 100 mM, pH range between 8.5 and 9.5, KCI in a range of concentrations between 480 and 560 mM and MgCl₂, in a range of concentrations between 10 and 20 mM.

13. A kit to detect the presence and virulence of an *H*. *pylori* strain in different types of samples according to claim 9, wherein the Taq polymerase used for the sequence amplification has a concentration range of between 400 and 6000U.

14. A kit to detect the presence and virulence of an *H*. *pylori* strain in different types of samples according to claim 9, wherein the concentration range of the dinucleotidees is between 15 and 30mM.

15. A kit to detect the presence and virulence of an *H*. *pylori* strain in different types of samples according to claim 9, wherein the primers used have the following particularities:
a. *H. pylori* identification gene, 16 *DNAr H. pylori:* Forward 5' CTG GAG AGA CTA AGC CCT CCA 3'; Reverse 5' CAT TAC TGA CGC TGA TTG 3';
b. Virulence genes:
i. *cagA* Forward 5' TCA GA AAT TTG GGG ATC AGC 3';
Reverse 5'GGG GAA CTG GTT CTT GAT TG 3';
ii. *vacAm alelle1* Forward 5'ATT TGG TCC GAG GTG GGA AAG T 3';
Reverse 5'GCT AGG CGC TCT TTG AAT TGC T 3';
iii. Gene *dupA,* Forward 5' ACA AGG ACG ATT GAG CGA TGG GAA 3';
Reverse 5' TGG CTA GTT TGA GGT CTT AGG CGT 3'';
c. DNA quality determination gene 16S DNAr Eub; Forward 5' GCA CAA GCG GTG GAG CAT GTG G 3'; Reverse 5' GCC CGG GAA CGT ATT CAC CG 3''.

16. A kit to detect the presence and virulence of an *H. pylori* strain in different types of samples according to claim 9, wherein the primers used have a concentration of between 1 and 20 pmol/l.

17. A kit to detect the presence and virulence of an *H. pylori* strain in different types of samples according to claim 9, wherein all the reactives in the kit exist in two physical forms:
a. dehydrated, sterile and lyophilized, or
b. liquid and sterile.

18. A kit to detect the presence and virulence of an *H*. *pylori* strain in different types of samples according to claim 9, wherein the amplification controls of the sequences of interest are:
a. positive control: DNA from *H pylori* strain ATCC43504,
b. negative control: human DNA.

19. A kit to detect the presence and virulence of an *H*. *pylori* strain in different types of samples according to claim 9, wherein the primers are previously standardized:
a. *H. pylori* identification gene, *16 DNAr H. pylori:* Forward 5' CTG GAG AGA CTA AGC CCT CCA 3'; Reverse 5' CAT TAC TGA CGC TGA TTG 3';
b. virulence genes:
i. *cagA* Forward 5' TCA GA AAT TTG GGG ATC AGC 3';
Reverse 5'GGG GAA CTG GTT CTT GAT TG 3';
ii. *vacAm alelle1* Forward 5'ATT TGG TCC GAG GTG GGA AAG T 3';
Reverse 5'GCT AGG CGC TCT TTG AAT TGC T 3';
iii. *Gene dupA,* Forward 5' ACA AGG ACG ATT GAG CGA TGG GAA 3';
Reverse 5' TGG CTA GTT TGA GGT CTT AGG CGT 3'';
c. DNA quality determination gene 16S DNAr Eub; Forward 5' GCA CAA GCG GTG GAG CAT GTG G 3', Reverse 5' GCC CGG GAA CGT ATT CAC CG 3''.

20. A kit to detect the presence and virulence of an *H*. *pylori* strain in different types of samples according to claim 9, wherein the virulence genes, specie identification genes, and DNA extraction quality genes are amplified in unison.

21. A kit to detect the presence and virulence of an *H*. *pylori* strain in different types of samples according to claim 9, wherein because the amplification is carried out with a volume of 25 µl, a temperature range between 58 and 65 °C; a cycle range between 35 and 45, using amplification bead support.

22. A kit to detect the presence and virulence of an *H*. *pylori* strain in different types of samples according to claim 9, wherein the kit can be used with a concentration of 1 ng of DNA.

23. A kit to detect the presence and virulence of an *H. pylori* strain in different types of samples according to claim 9, wherein the kit includes primers that allow the determination of the quality of the DNA extracted.

24. A kit to detect the presence and virulence of an *H*. *pylori* strain in different types of samples according two claim 9, wherein the kit determines the presence and virulence of different *H*. *pylori* strains, in diverse samples, including body fluids, tissue or feces.

25. A kit to detect the presence and virulence of an *H. pylori* strain in different types of samples according to claim 9, wherein the kit allows the identification of virulence genes *cagA, vacAm alelle1,* and dupA gene.

26. A kit to detect the presence and virulence of an *H. pylori* strain in different types of samples according to claim 9, wherein the kit allows the identification of *H*. *pylori* using the gene *16S DNAr.*

27. A kit to detect the presence and virulence of an *H. pylori* strain in different types of samples according to claim 9, wherein the kit allows the determination of DNA quality, using visualization of the gene 16S DNAr Eub.

28. A kit to detect the presence and virulence of an *H. pylori* strain in different types of samples according to claim 9, wherein the kit can include ultra pure water without nucleases.

29. A kit to detect the presence and virulence of an *H. pylori* strain in different types of samples according to claim 9, wherein the kit contains a users' manual.
